# DEMANDE DE BREVET EUROPEEN

(11) **EP 1 437 154 A2**
(43) Date de publication de la demande: **14.07.2004**
(21) Numéro de dépôt: 04290033.2
(22) Date de dépôt: 07.01.2004
(51) Int. Cl.: A61M 25/01

(54) **Dispositif de commande manuelle d'un guide chirurgical**

(30) Priorité: 07.01.2003 FR 0300110
(71) Demandeur: SOCIETE D'ETUDES ET APPLICATIONS TECHNIQUES ( S.E.D.A.T.), 69450 Irigny (FR)
(72) Inventeur: Denolly, Pascal, 38200 Jardin (FR)
(74) Mandataire: Blot, Philippe Robert Emile

(57) **Abrégé**

Ce dispositif de commande manuelle (2) comporte un corps (10) délimitant intérieurement un conduit (12) de passage d'un guide chirurgical (2). Pour assurer le blocage axial et angulaire du guide par rapport au corps, le dispositif comporte deux langues (14) reliées au corps (10). A la façon d'une genouillère, la partie courante (46) de chaque langue est élastiquement déformable suivant une direction transversale au conduit de passage entre une position de blocage du guide chirurgical (2) et une position de libération de ce guide.

Application à la manoeuvre d'un guide chirurgical (2).

## Description

La présente invention concerne un dispositif de commande manuelle d'un guide chirurgical, du type comportant un corps délimitant intérieurement un conduit de passage du guide chirurgical, et des moyens de blocage du guide chirurgical par rapport au corps.

Un tel dispositif de commande est utilisé pour faciliter le maniement d'un guide mis en place lors d'interventions en angioplastie et en angiographie. Un tel guide est constitué d'un fil souple de grande longueur, pouvant atteindre 2 m et présentant un diamètre très petit, compris généralement entre 0,25 et 1,15 mm.

Le dispositif de commande manuelle monté sur le guide permet, lors d'une intervention, de pousser le guide, de le tirer et de le tourner sur lui-même, après que l'extrémité de celui-ci ait été introduite dans le corps humain et notamment à l'intérieur d'un conduit vasculaire. Le déplacement du guide est facilité puisque le dispositif de commande manuelle constitue un organe solidaire du guide, dont les dimensions facilitent la prise en main par rapport à une action directe sur le guide qui est trop fin pour être saisi facilement.

Des dispositifs de commande manuelle de guide sont déjà connus et couramment utilisés. Ceux-ci comportent d'une part un corps traversé par un conduit de passage du guide, et d'autre part des moyens de blocage axial et angulaire du guide par rapport au corps.

Dans certains dispositifs, ces moyens de blocage comportent une bague rapportée, adaptée pour être vissée sur le corps en comprimant radialement le guide, assurant l'immobilisation du guide par rapport au corps. De tels dispositifs sont relativement complexes et d'un coût de fabrication élevé puisqu'ils comportent plusieurs pièces, ces dernières devant être ajustées avec précision afin de permettre un vissage efficace.

Dans d'autres dispositifs de commande manuelle, les moyens de blocage du guide comportent un coulisseau monté sur le corps, assurant l'immobilisation du guide chirurgical par effet de coin. Le coulisseau est à cet effet sollicité par une rampe formant came ménagée sur le corps. Ces dispositifs sont également complexes à réaliser, puisqu'il est nécessaire de mettre en oeuvre plusieurs éléments et des moyens de guidage précis des éléments les uns par rapport aux autres, afin d'assurer un fonctionnement satisfaisant.

Le document WO-97/18850 décrit quant à lui un dispositif du type précité, comportant un corps tubulaire qui délimite un conduit de réception d'un guide chirurgical et sur lequel est rapporté extérieurement un manchon déplaçable en translation par rapport au corps. Le manchon est pourvu à son extrémité enfilée sur le corps tubulaire d'une langue rigide dont une extrémité est libre et dont l'autre est venue de matière avec le manchon en formant une articulation. En combinant une sollicitation sur cette langue suivant une direction transversale au manchon et un mouvement de translation du manchon par rapport au corps, le chirurgien peut introduire la langue dans le corps et provoquer le serrage bloquant du guide entre la langue et la paroi interne du conduit délimité par le corps. Des crochets élastiques assurent la retenue en translation du manchon par rapport au corps.

Ce dispositif est également complexe à réaliser et à manipuler puisqu'il repose sur la coopération du corps, du manchon mobile et de la langue articulée. De plus, les crochets de retenue du manchon sont d'utilisation peu pratique, le chirurgien devant développer un effort important d'écartement du manchon vis-à-vis du corps pour vaincre la retenue de ces crochets lors de la libération du guide.

De manière plus générale, l'ensemble des dispositifs connus sont d'un maniement relativement délicat pour le chirurgien, puisque la libération du guide chirurgical et/ou son immobilisation nécessitent généralement ses deux mains.

L'invention a pour but de fournir un dispositif de commande manuelle pour guide chirurgical ayant un faible coût de fabrication et permettant un usage simple, notamment à une seule main lors de l'immobilisation et de la libération de ce guide.

A cet effet, l'invention a pour objet un dispositif de commande manuelle d'un guide chirurgical du type précité, caractérisé en ce que les moyens de blocage comportent au moins une langue comprenant une partie courante reliée au corps depuis une première extrémité, laquelle partie courante est élastiquement déformable suivant une direction transversale au conduit de passage entre une configuration de blocage du guide chirurgical par la langue et une configuration de libération de ce guide, la langue étant agencée de telle sorte que la commutation entre les deux configurations de blocage et de libération sont obtenues par franchissement d'une configuration de point mort instable de la partie courante de la langue depuis laquelle cette partie courante se déforme spontanément vers l'une ou l'autre des configurations de blocage et de libération.

Suivant d'autres caractéristiques de ce dispositif, prises isolément ou selon toutes les combinaisons techniquement possibles :
- la ou chaque langue est venue de manière avec le corps ;
- la première extrémité de la ou chaque langue, forme une charnière pour l'articulation de la langue par rapport au corps lorsque la partie courante de la langue correspondante est déformée entre ses configurations de blocage et de libération ;
- la ou chaque langue comprend un mors d'appui sur le guide chirurgical disposé au moins partiellement dans le conduit de passage ;
- le ou chaque mors présente une surface de came adaptée pour coopérer avec une surface de contre-came délimitée par la surface du conduit de passage ;
- le ou chaque mors est prévu à une extrémité libre de la ou chaque langue ;
- la ou chaque langue présente une forme générale d'arche dans ses configurations de blocage et de libération, la courbure de la ou chaque langue étant inversée entre la configuration de blocage et la configuration de libération ;
- chaque langue est souple dans sa partie courante ;
- la ou chaque langue comporte une surface d'appui digital ;
- ce dispositif comporte, pour la ou chaque langue, un doigt de libération de la langue associée, lequel doigt déplaçable par rapport au corps et présente une surface d'actionnement de la partie courante de la langue associée ;
- le ou chaque doigt rigide est venu de matière avec le corps ;
- le ou chaque doigt de libération est déplaçable par rapport au corps suivant une direction transversale à la direction générale de déformation de la langue associée ; et
- les moyens de blocage comportent deux langues situés sur deux côtés opposés du corps.

L'invention sera mieux comprise à la lecture de la description qui va suivre, donnée uniquement à titre d'exemple et faite en se référant aux dessins sur lesquels :
- la figure 1 est une vue en perspective d'un dispositif de commande selon l'invention, représenté seul ;
- la figure 2 est une vue en perspective du dispositif de la figure 1 dans sa configuration d'introduction ou de libération d'un guide chirurgical, et à l'intérieur duquel est enfilé un tel guide ;
- la figure 3 est une vue en coupe longitudinale du dispositif représenté sur la figure 2 ;
- la figure 4 est une vue en perspective du dispositif de la figure 1 dans sa configuration de blocage du guide ; et
- la figure 5 est une coupe longitudinale du dispositif représenté sur la figure 4.

Le dispositif 1 selon l'invention, représenté aux figures, est destiné à la commande manuelle d'un guide chirurgical 2. Il présente une forme générale allongée sensiblement cylindrique d'axe X-X. Il comporte, d'une part, un corps 10 traversé de part en part axialement par un conduit interne 12 et, d'autre part, deux langues souples 14 de blocage du guide chirurgical par rapport au corps. Ces deux langues s'étendent le long du corps, de façon diamétralement opposée. Les langues 14 sont venues de matière avec le corps 10.

Plus précisément, le corps 10 comporte un tronçon de préhension 18 à la surface extérieure duquel sont ménagés des bourrelets longitudinaux 20 facilitant le maniement du dispositif. Le tronçon de préhension 18 est prolongé par un tronçon 22 de blocage du guide chirurgical 2. Ce tronçon 22 est délimité par deux poutres 23 parallèles à l'axe X-X, disposées symétriquement de part et d'autre de cet axe. Ces poutres définissent latéralement deux méplats 24. Les poutres sont prolongées par une tête tubulaire 26 les reliant.

Les poutres 23 sont traversées de deux fentes longitudinales 28. Ces fentes sont diamétralement opposées. Les fentes 28 s'étendent dans un plan diamétral du tronçon 22 parallèle aux méplats 24.

Elles reçoivent chacune un doigt 30, venu de matière avec la tête 26 en formant une charnière 31 déformable élastiquement. Les faces 32 de ces doigts en regard l'une de l'autre, c'est-à-dire les faces dirigées vers le conduit 12, sont planes. Au repos, c'est-à-dire en dehors de toute contrainte appliquée sur les doigts, elles s'étendent en retrait dans les fentes 28. Les faces externes 33 des doigts 30 présentent un profil en saillies et en creux, en vue de faciliter l'action manuelle sur ces doigts. Ces faces externes font saillie hors des fentes 28.

Le conduit 12 s'étend sur toute la longueur du corps 10. Il comporte successivement :
- un premier tronçon 34 qui s'étend sur toute la longueur du tronçon de préhension 18 et qui présente une section transversale sensiblement rectangulaire ;
- un deuxième tronçon 36 délimité entre les poutres 33 suivant le tronçon de blocage 22 ; ce tronçon 36 débouche sur l'extérieur au niveau des méplats 24 ; et
- un troisième tronçon 38 délimité intérieurement par la tête 26.

Ce tronçon 38 présente notamment, à l'extrémité libre de la tête 26, une section transversale progressivement croissante vers l'extérieur formant un cône de centrage 40 facilitant l'introduction du guide chirurgical 2.

Chaque langue souple 14 est reliée au corps 10 depuis une première extrémité 42 constituant une région de liaison 44 formant charnière, déformable élastiquement.

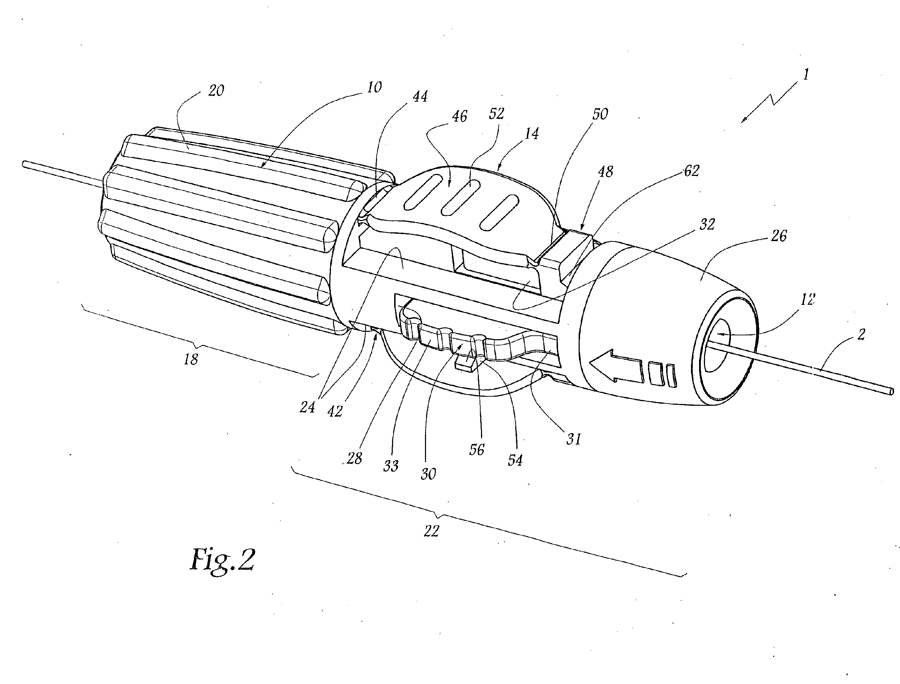

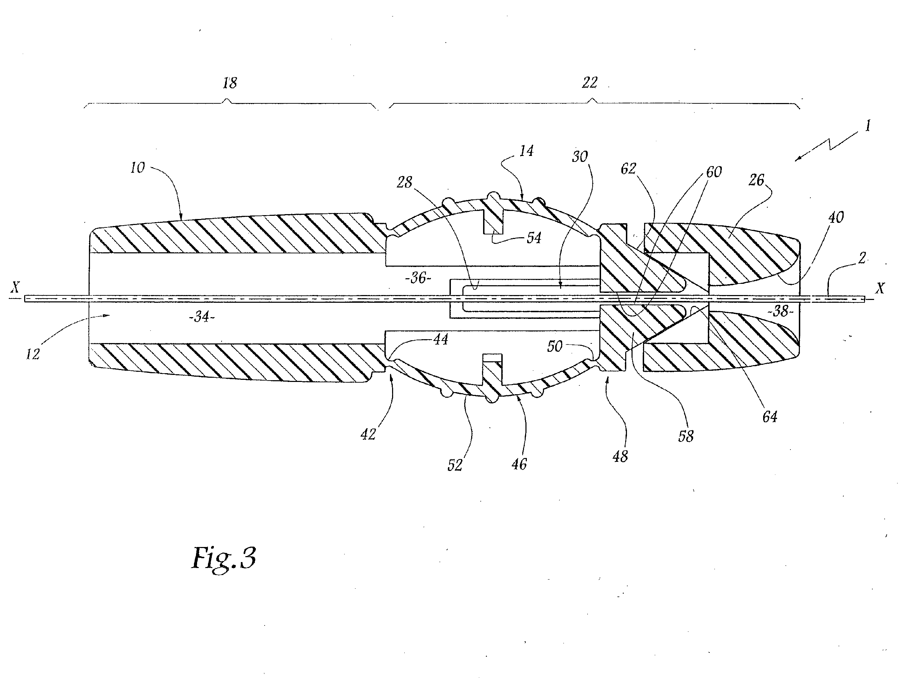

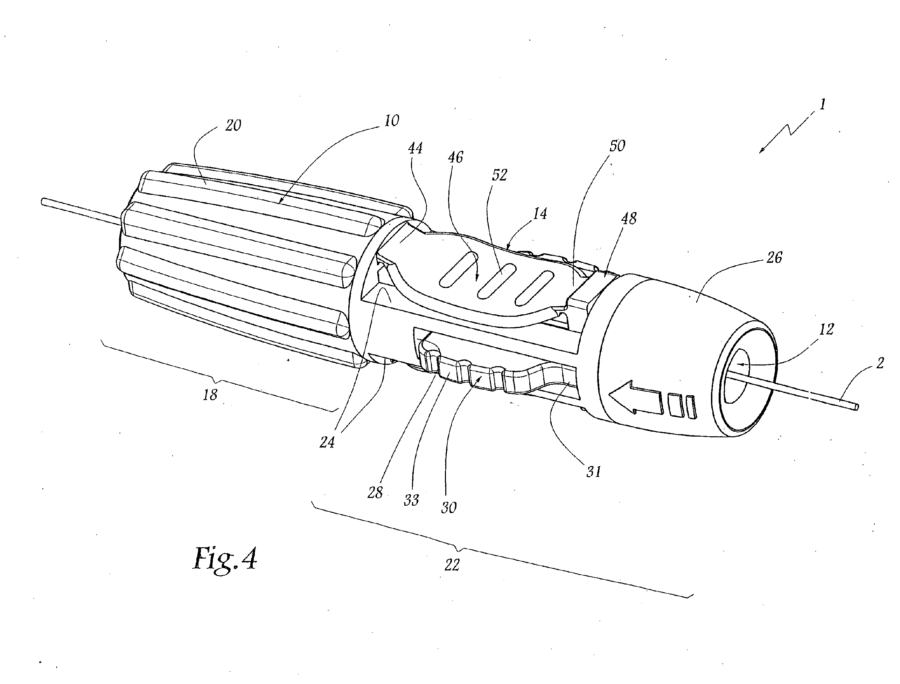

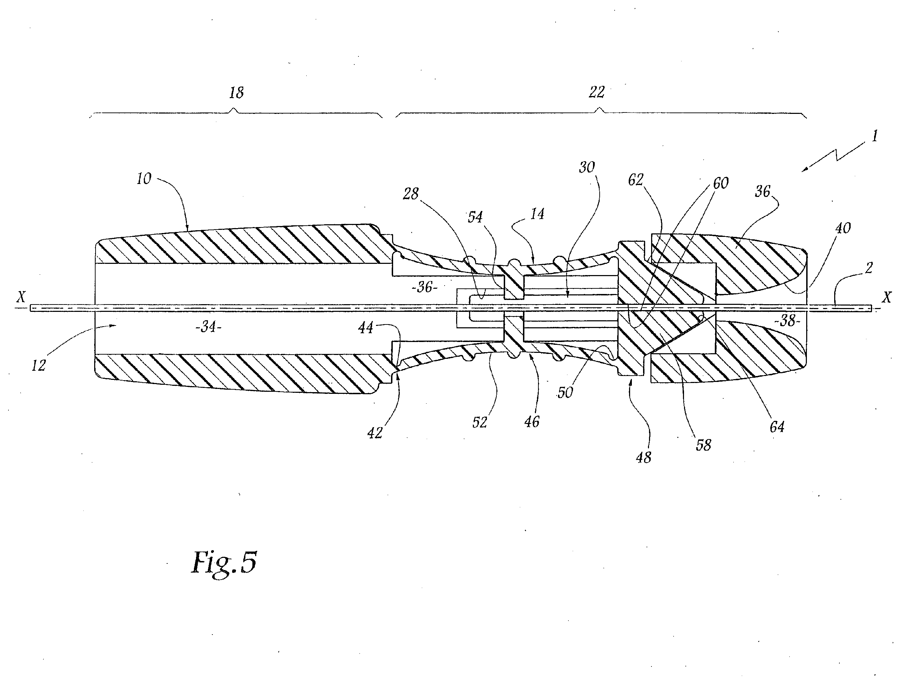

Elle comporte une partie courante 46 constituée d'une lame de matière plastique formant genouillère, déformable élastiquement suivant une direction transversale à l'axe X-X, entre une configuration d'introduction ou de libération du guide chirurgical 2, représentée sur les figures 2 et 3 et dans laquelle la lame 46 est convexe, sa partie médiane faisant saillie vers l'extérieur, et une configuration de blocage du guide par la langue, représentée sur les figures 4 et 5 et dans laquelle la lame 46 est concave, sa partie médiane étant rentrée vers l'axe X-X.

Un coin d'extrémité 48 prolonge la lame 46 en étant reliée à celle-ci par une région de liaison 50 formant charnière.

Afin de faciliter la déformation des régions de liaison 44 et 50 de chaque langue, ces régions sont situées en regard du méplat correspondant 24, ce dernier étant ménagé à une distance suffisante des extrémités 42 et 48 de la langue.

La face externe de la lame 46 de chaque langue présente un profil en saillies et en creux et 52 facilitant la sollicitation manuelle de la langue correspondante. La face interne de chacune de ces lames 46 est quant à elle pourvue en son milieu d'un ergot saillant 54 définissant une surface de came 56 formant une rampe inclinée par rapport au reste de la face interne. La surface de came 56 est généralement parallèle à l'axe X-X.

Le coin d'extrémité 48 de chaque langue 14 forme un mors 58 d'appui sur le guide 2. Il est de forme générale pyramidale. Chaque mors 58 délimite une surface d'appui plane 60 orientée vers l'intérieur du tronçon de conduit 36, ainsi qu'une surface plane 62 inclinée par rapport à la surface 60. Cette surface 60 forme une surface de came propre à coopérer avec une surface de contre-came 64 ménagée dans une chambre du tronçon 38 qui s'étend entre le tronçon 36 et l'extrémité de petit diamètre du cône de centrage 40.

Les surfaces de came 62 et de contre-came 64 sont planes et s'étendent parallèlement l'une à l'autre. Elles définissent un angle aigu avec l'axe X-X, ces surfaces convergeant vers l'axe X-X suivant la direction de la tête 26.

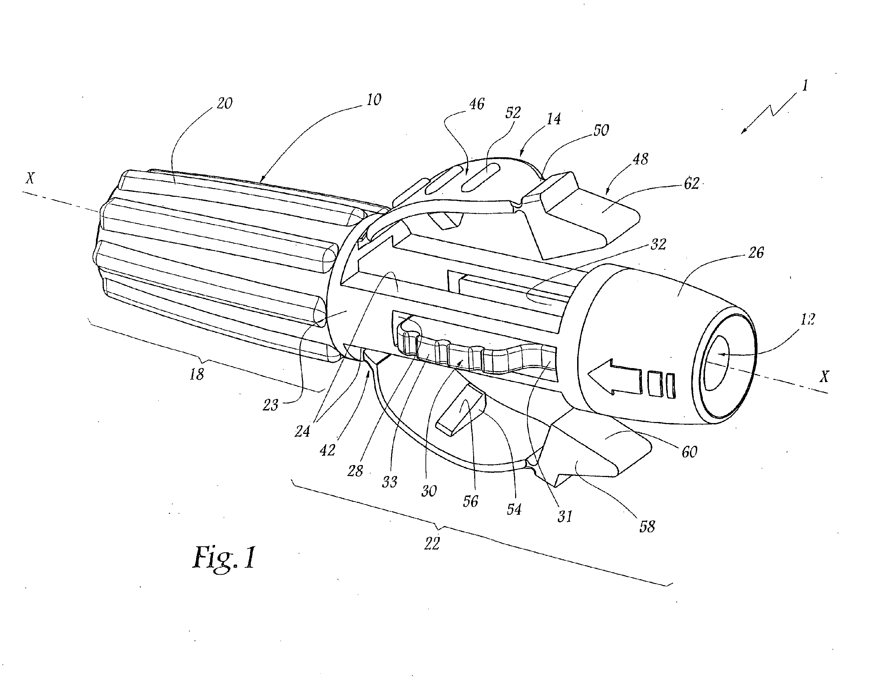

Comme représenté sur les figures, les langues 14 et les doigts 30 sont tous venus de matière avec le corps 10. A cet effet, le dispositif de commande manuelle 1 est réalisé par injection de matière plastique, en une seule fois, dans un moule de forme adaptée. A la sortie de ce moule, le dispositif se présente suivant la configuration représentée sur la figure 1.

Dans cette configuration, les coins 48 sont en dehors du conduit 12, ce qui permet un démoulage aisé du dispositif.

Lorsque le dispositif de commande manuelle 1 est en fonctionnement, c'est-à-dire lorsque les langues souples 14 sont soit en configuration de libération du guide, soit en configuration de blocage de ce guide, les mors 58 sont en partie reçus à l'intérieur du conduit 12 de sorte que les surfaces 60 des mors s'étendent sensiblement parallèlement l'une à l'autre, suivant un plan parallèle aux méplats 24, et que les mors 58 sont coincés à l'intérieur de la tête 36.

Plus précisément, lorsque les langues sont en configuration d'introduction ou de libération du guide, les surfaces 60 sont distantes l'une de l'autre d'une hauteur supérieure au diamètre du guide 2 et les surfaces de came 62 sont en appui contre les surfaces de contre-came associées 64. Les surfaces 60 sont maintenues écartées l'un de l'autre en raison de la tendance des régions de liaison élastique 44 et 50 à reprendre leurs états initiaux de moulage représentés sur la figure 1. Cette position d'introduction ou de libération du guide est stable.

De plus, lorsque les langues souples sont dans cette configuration d'introduction ou de libération du guide chirurgical 2, les lames 46 sont à même d'être déformées dans leur partie courante par un effort manuel de serrage appliqué transversalement à l'axe X-X, de préférence au milieu de ces lames courantes. Par déformation, ces lames atteignent une configuration dite « de point mort » dans laquelle elles sont sensiblement planes et s'étendent sensiblement parallèlement aux méplats 24. Cette configuration constitue un équilibre instable.

Lors de la déformation des lames 46, les mors 58 sont repoussés vers l'extrémité libre de la tête 26 suivant la direction de l'axe X-X. Sous l'action des surfaces de came/contre-came 62, 64, les mors 58 sont repoussés radialement vers l'axe X-X, provoquant ainsi le serrage du guide chirurgical 2 entre les mors. Les mors sont alors coincés entre le guide chirurgical et les surfaces de contre-came par effet de coin.

Lors de la poursuite de la déformation des lames 26, la courbure de celles-ci s'inverse jusqu'à ce qu'elles atteignent une forme concave comme illustré aux figures 4 et 5. Cette configuration est stable et peut même être obtenue sans action manuelle par action élastique des lames après franchissement de la configuration de point mort.

Dans cette configuration de blocage, les faces internes 32 des doigts s'étendent en regard des surfaces inclinées 56 des ergots.

Le dispositif de commande manuelle 1 s'utilise de la façon suivante :

Initialement, le dispositif est dans sa configuration de moulage, illustrée sur la figure 1. Par déformation des lames 46 et des régions de liaison 44 et 50 des langues souples 14, les mors 58 sont amenés en partie à l'intérieur du conduit 12, ce qui confère au dispositif une configuration fonctionnelle d'introduction du guide chirurgical 2.

Ce guide est alors introduit à l'intérieur du dispositif 1, de préférence par le cône de centrage 40 ménagé à l'extrémité libre du tronçon de blocage 22.

Après mise en place du dispositif 1 autour du guide chirurgical dans la région souhaitée comme sur les figures 2 et 3, le chirurgien exerce avec les doigts d'une de ses mains un effort transversal à l'axe X-X sur les faces externes des lames 46. Chaque lame se déforme alors à la façon d'une genouillère, en passant dans un premier temps de sa configuration d'introduction du guide à sa configuration de point mort, puis dans un deuxième temps de sa configuration de point mort jusqu'à sa configuration de blocage du guide.

Le dispositif 1 se retrouve alors dans sa configuration de blocage des figures 4 et 5. Le guide chirurgical 2 peut ainsi être facilement manipulé par le chirurgien, qui maintient le dispositif de commande entre ses doigts, en le manipulant notamment par son tronçon de préhension 18.

Lorsque le chirurgien souhaite faire coulisser le dispositif de commande manuelle 1 le long du guide, ou déplacer angulairement le dispositif de commande par rapport au guide, il exerce manuellement une force d'appui sur les doigts 30 de manière à provoquer leur basculement autour de la zone de liaison 31 les reliant au corps 10. Les faces internes 32 de ces doigts viennent alors en appui contre les surfaces inclinées 56 des ergots. Par effet de came sur les surfaces 56, les doigts 30 créent un effort sur les lames 46 transversalement à l'axe X-X dirigé vers l'extérieur du dispositif.

Les langues 14 passent alors de leur configuration de blocage à leur configuration de point mort, au-delà de laquelle elles reprennent élastiquement leur configuration de libération du guide des figures 2 et 3.

La réalisation en une seule pièce du dispositif de commande manuelle selon l'invention réduit le coût de fabrication de ce dispositif et augmente sa fiabilité. De plus, sa manipulation est extrêmement simple, le guide chirurgical pouvant passer de sa configuration de blocage à sa configuration d'introduction ou de libération par simple sollicitation manuelle sur les langues souples ou sur les doigts de déblocage. De telles sollicitations digitales s'effectuent de manière naturelle pour le chirurgien, avec une seule main, contrairement au vissage d'un organe de blocage et/ou au déplacement axial d'un tel organe par rapport au corps qui nécessitent une agilité importante.

De plus, la pression exercée par les moyens de blocage du guide chirurgical est fixée par le coincement des mors 58 dans la tête 26. Cette pression, liée directement à la force constante de maintien des langues souples dans leur configuration stable de blocage, est constante et ne dépend pas de l'effort appliqué par le chirurgien, contrairement à certains dispositifs de l'état de la technique où l'effort de vissage ou de coincement par déplacement axial d'un organe de commande correspondant dépend de l'action du chirurgien. Ainsi, tout risque de mauvais blocage par un effort de serrage ou de coincement insuffisant ou de détérioration du guide par un effort excessif est évité.

En variante au dispositif décrit ci-dessus, une des deux langues souples 14 est supprimée, ainsi que son doigt de blocage associé 30. Dans ce cas, le guide chirurgical est coincé entre le mors de la langue restante et une paroi sensiblement plane en regard, délimitée par le conduit 12.

## Revendications

1. Dispositif de commande manuelle d'un guide chirurgical (2), du type comportant un corps (10) délimitant intérieurement un conduit (12) de passage du guide chirurgical (2), et des moyens de blocage du guide chirurgical par rapport au corps, **caractérisé en ce que** les moyens de blocage comportent au moins une langue (14) comprenant une partie courante (46) reliée au corps (10) depuis une première extrémité (42), laquelle partie courante est élastiquement déformable suivant une direction transversale au conduit de passage (12) entre une configuration de blocage du guide chirurgical par la langue (14) et une configuration de libération de ce guide, la langue (14) étant agencée de telle sorte que la commutation entre les deux configurations de blocage et de libération sont obtenues par franchissement d'une configuration de point mort instable de la partie courante (46) de la langue depuis laquelle cette partie courante (46) se déforme spontanément vers l'une ou l'autre des configurations de blocage et de libération.

2. Dispositif suivant la revendication 1, **caractérisé en ce que** la ou chaque langue (14) est venue de manière avec le corps (10).

3. Dispositif suivant la revendication 1 ou 2, **caractérisé en ce que** la première extrémité (42) de la ou chaque langue (14), forme une charnière pour l'articulation de la langue (14) par rapport au corps lorsque la partie courante (46) de la langue correspondante est déformée entre ses configurations de blocage et de libération.

4. Dispositif suivant l'une quelconque des revendications précédentes, **caractérisé en ce que** la ou chaque langue (14) comprend un mors (58) d'appui sur le guide chirurgical (2) disposé au moins partiellement dans le conduit de passage (12).

5. Dispositif suivant la revendications 4, **caractérisé en ce que** le ou chaque mors (58) présente une surface de came (62) adaptée pour coopérer avec une surface de contre-came (64) délimitée par la surface du conduit de passage (12).

6. Dispositif selon la revendication 4 ou 5, **caractérisé en ce que** le ou chaque mors (58) est prévu à une extrémité libre de la ou chaque langue (14).

7. Dispositif selon l'une quelconque des revendications précédentes, **caractérisé en ce que** la ou chaque langue (14) présente une forme générale d'arche dans ses configurations de blocage et de libération, la courbure de la ou chaque langue (14) étant inversée entre la configuration de blocage et la configuration de libération.

8. Dispositif selon l'une quelconque des revendications précédentes, **caractérisé en ce que** chaque langue (14) est souple dans sa partie courante (46).

9. Dispositif suivant l'une quelconque des revendications précédentes, **caractérisé en ce que** la ou chaque langue (14) comporte une surface d'appui digital (52).

10. Dispositif suivant l'une quelconque des revendications dépendantes, **caractérisé en ce qu'**il comporte, pour la ou chaque langue (14), un doigt (30) de libération de la langue (14) associée, lequel doigt (30) déplaçable par rapport au corps (10) et présente une surface (32) d'actionnement de la partie courante (46) de la langue associée.

11. Dispositif suivant la revendication 10, **caractérisé en ce que** le ou chaque doigt rigide (30) est venu de matière avec le corps (10).

12. Dispositif suivant la revendication 10 ou 11, **caractérisé en ce que** le ou chaque doigt de libération (30) est déplaçable par rapport au corps suivant une direction transversale à la direction générale de déformation de la langue (14) associée.

13. Dispositif suivant l'une quelconque des revendications précédentes, **caractérisé en ce que** les moyens de blocage comportent deux langues (14) situés sur deux côtés opposés du corps (10).
